# EUROPÄISCHE PATENTSCHRIFT

(11) **EP 1 952 757 B1**
(45) Veröffentlichungstag und Bekanntmachung des Hinweises auf die Patenterteilung: **22.04.2020**
(21) Anmeldenummer: 08001758.5
(22) Anmeldetag: 31.01.2008
(51) Int. Cl.: A61B 5/145, G16H 10/00, G16H 40/63

(54) **Datenverarbeitungsvorrichtung zur Verarbeitung von Messwerten**
Data processing device for processing measurements
Dispositif de traitement des données destiné au traitement de valeurs de mesure

(30) Priorität: 31.01.2007 EP 07002063
(43) Veröffentlichungstag der Anmeldung: 06.08.2008
(73) Patentinhaber: F. Hoffmann-La Roche AG, 4070 Basel (CH); Roche Diabetes Care GmbH, 68305 Mannheim (DE)
(72) Erfinder: Kintzig, Hans, 67311 Tiefenthal (DE); Porsch, Ulrich, 69469 Weinheim (DE); Blatt, Christian, 69239 Neckarsteinach (DE)
(74) Vertreter: Glawe, Delfs, Moll

(56) Entgegenhaltungen:
- EP-A- 0 607 727
- EP-A- 1 369 688
- EP-A- 1 372 102
- EP-A- 1 674 975
- EP-A- 1 728 470
- EP-A- 1 769 963
- WO-A-2007/005170
- US-A1- 2004 016 802
- US-A1- 2005 201 585
- US-B1- 6 205 427

## Beschreibung

Die vorliegende Erfindung betrifft eine Datenverarbeitungsvorrichtung zur Verarbeitung von Messwerten und ein Verfahren zum Betrieb einer Datenverarbeitungsvorrichtung. Insbesondere betrifft die vorliegende Erfindung eine Datenverarbeitungsvorrichtung zur akustischen Ausgabe von medizinisch relevanten Daten.

Im medizinischen Bereich ist es bekannt, tragbare Patientengeräte zum Sammeln von Patientendaten einzusetzen. Häufig sind diese tragbaren Geräte mit zentralen Datenverarbeitungsgeräten verbunden, in denen eine Überwachung, Auswahl, Analyse usw. der Daten entweder von medizinischem Personal, Ärzten oder auch automatisiert vorgenommen wird. Derartige Geräte werden u.a. eingesetzt, um Blutzuckerwerte von Diabetikern zu sammeln und zu überwachen. Aus der EP 1 559 364 A1 ist bspw. ein drahtloses Diabetesüberwachungssystem bekannt, bei dem den Patienten nach Übermittlung seiner Blutzuckerwerte an eine Zentrale Verhaltensanweisungen über ein mobiles Telefon mitgeteilt werden. Ein weiteres vergleichbares System ist aus der US 2005/0089150 A1 bekannt, bei dem über Telefon und tragbare Geräte eine interaktive Anweisung eines Nutzers/Patient mittels Spracherkennungssystemen und softwareerzeugten Anweisungen an den Nutzer erfolgt.

An Diabetes Mellitus erkrankte Personen müssen bestrebt sein, ihren Blutzuckerwert stets in einem bestimmten Bereich zu halten. Wird der angestrebte Bereich überschritten, muss Insulin gespritzt werden. Wird der angestrebte Bereich unterschritten, muss z.B. Zucker oral (über die Nahrung oder ein Getränk) zugeführt werden. Wird der angestrebte Bereich über eine längere Zeit überschritten, besteht die Gefahr von ernsthaften gesundheitlichen Komplikationen, wie Blindheit, Nierenschädigung, Absterben von Gliedmaßen oder Neuropathie. U.a. bei kurzfristigem, starkem Überschreiten des Bereiches kann es zu Übelkeit, Schwindel, Schweißausbrüchen und sogar zu Zuständen der Verwirrtheit kommen. Bei einem kurzzeitigen Unterschreiten des angestrebten Bereichs kann es ebenfalls zu Übelkeit, Schwindel, Schweißausbrüchen, Verwirrtheit und - im schlimmsten Falle - zum Tode des Diabetikers kommen. Daher ist es dringend geboten, dass ein Diabetiker jederzeit seinen Blutzuckerstatus kennt und ggf. eigenständig geeignete Maßnahmen einleiten kann, um ein Über- oder Unterschreiten des Blutzuckerwerts aus dem angestrebten Bereich zu vermeiden. Hierzu werden bereits seit geraumer Zeit Blutzuckermessgeräte verwendet, wie sie bspw. aus der DE 10 2004 057 503 A1 bekannt sind und von der Anmelderin unter der eingetragenen Marke Accu-Chek® vertrieben werden. Im Idealfall handhabt der Diabetiker die Messung des Blutzuckerwerts und sich daraus ergebende Maßnahmen in Eigenverantwortung.

Der Blutzuckerwert unterliegt starken Schwankungen in Abhängigkeit von den Insulingaben (in der Regel werden unterschiedlich wirkende Insuline gleichzeitig verwendet), von den zugeführten Zuckermengen und anderen physiologisch auf den Zuckerstoffwechsel wirkenden Nahrungs- und Genussmitteln. Ebenfalls auf den Zuckerstoffwechsel wirken körperliche Bewegung, Stress, Krankheit u.v.a.m. Da nicht jeder Organismus in gleicher Weise auf diese physiologischen Größen reagiert, muss jeder Diabetiker seine eigenen physiologischen Reaktionen kennenlernen. Hierzu ist das Führen eines Diabetes-Tagebuchs unerlässlich. Anhand der Eintragungen in ein solches schriftlich geführtes Tagebuch kann der Diabetiker in der Historie seiner Eintragungen ähnliche Situationen suchen und mit der aktuellen Situation vergleichen, um dann entsprechende Maßnahmen zur Korrektur des Stoffwechsels einzuleiten. Er ist aufgrund der Aufzeichnungen in der Lage, erfolgreiche Korrekturen des Stoffwechsels zu wiederholen oder durch entsprechende Anpassungen der Stellglieder die physiologischen Situationen besser zu korrigieren als in der Vergangenheit, wenn in einer ähnlichen Situation eine Korrektur nicht zum gewünschten Erfolg geführt hat. Daraus ergibt sich, wie bereits angeführt, eine dringende Notwendigkeit für jeden Diabetiker, ein derartiges Tagebuch zu führen, in dem alle Parameter bzw. Stellglieder des Stoffwechsel-Regelkreises vermerkt werden.

Ca. 80 % aller blinden Diabetiker haben ihre Blindheit durch Diabetes, d.h. der Blutzucker dieser Menschen war über eine längere Zeit nicht richtig eingestellt, was zur Erblindung führte. Aufgrund ihrer Erblindung ist es diesen Diabetikern verwehrt, selbst ein Tagebuch wie voranstehend beschrieben zu führen, und sie waren bisher nicht in der Lage, eigenständig eine Insulintherapie durchzuführen. Zwar ist eine Betreuung durch andere Personen möglich, jedoch zeigen Erfahrungswerte, dass dann die Blutzuckereinstellung des Patienten schlechter ist als bei einer eigenverantwortlichen Einstellung des Blutzuckers, d.h. mit einer eigenverantwortlichen Einstellung des Blutzuckers verringert sich die Gefahr von weiteren gesundheitlichen Komplikationen.

Die Gruppe der blinden und sehbehinderten Diabetiker hat somit ein großes Interesse daran, selbst ein Tagebuch führen und die darin festgehaltene Historie in Form von Daten selektieren zu können, um in kritischen Situationen geeignete Maßnahmen einzuleiten.

Aus der EP 1 369 688 A2 ist ein Glukosemessgerät für "sehende" Benutzer bekannt, das dem Benutzer verschiedene Sortier- und Navigationsmöglichkeiten über eine grafische Benutzerschnittstelle bietet. Bedienerleichterungen für Sehbehinderte oder blinde Benutzer sind nicht vorgesehen.

Die EP 0 607 727 A1 offenbart eine Datenverarbeitungsvorrichtung allgemeiner Art, bei der zur Vereinfachung der Benutzung durch eine blinde Person den einzelnen, auf einem Bildschirm zur Anzeige kommenden grafischen Objekten, die unterschiedliche Zustände haben können, jeweils ein akustisches Signal zugeordnet ist, das bei einer Statusänderung des grafischen Objekts ertönt.

Die EP 1 369 688 A2 offenbart ein Analyt-Testgerät, insbesondere ein Glukosemessgerät. Das bekannte Glukosemessgerät bietet verschiedene Möglichkeiten zum Sortieren und Navigieren für ausschließlich sehende Nutzer über eine grafische Benutzerschnittstelle (d.h. eine Anzeigeeinrichtung wie ein Kleinmonitor).

Aus der US 2005/0159656 A1 ist eine Vorrichtung zur Darstellung von Informationen betreffend nichtinvasiv erhaltenen Glukosegehaltwerten bekannt. Die bekannte Vorrichtung umfasst einen Anzeigeschirm und eine optionale Audio-Ausgabeeinheit wie einen Lautsprecher, zur Ausgabe von Lauten, Tönen oder auch synthetisierter Sprache. Der Benutzer kann aus einer Reihe von graphischen (optischen) und/oder akustischen Darstellungsoptionen auswählen.

Aus der US 6,205,427 B1 ist ein Sprachausgabegerät bekannt, das dazu dient, einer blinden Person geschriebene Texte "vorzulesen". Hierzu werden die Textdaten in eine Mehrzahl von Abschnitten zerlegt und jeder Abschnitt wird in ein Sprachsignal mit unterschiedlichen Geschwindigkeiten umgesetzt/konvertiert. Die Aufteilung der Textdaten erfolgt an vormarkierten Stellen abhängig davon, welche Abschnitte vom Nutzer ausgewählt und kombiniert werden, so dass der Text schnell und verständlich vorgelesen werden kann. Das Auslesen erfolgt mit zumindest zwei unterschiedlichen Geschwindigkeiten, getriggert durch den Benutzer über einen "normal reproduction key" und einen "fast feed reproduction key".

Erfindungsgemäß wird daher eine Datenverarbeitungsvorrichtung zur Verarbeitung von Messwerten von einem Analyse- oder Messgerät mit den Merkmalen des Anspruchs 1 sowie ein Verfahren zum Betrieb einer Datenverarbeitungsvorrichtung mit den Merkmalen des Anspruchs 13 vorgeschlagen. Das Analyse- oder Messgerät kann jedes Gerät sein, mit dem medizinisch relevante Daten bzw. Meßwerte erhoben werden, bspw. ein Blutzuckermessgerät.

Die Erfindung ermöglicht es auch blinden Diabetikern, in einfacher Art und Weise ein Tagebuch über die Entwicklung ihrer Blutzuckerwerte zu führen und aus der gespeicherten großen Datenmenge Datensätze zu selektieren, also insbesondere gleiche oder ähnliche Situationen aus der Vergangenheit aufzufinden und die physiologischen Reaktionen der vergangenen Situationen mit der aktuellen Situation zu vergleichen und geeignete Maßnahmen einzuleiten. Zur Messung der Blutzuckerwerte kann dabei ein konventionelles Blutzuckermessgerät dienen, dessen Benutzung auch blinden Diabetikern möglich ist. Mit dem konventionellen Blutzuckermessgerät erhobene Messwerte werden mittels der Dateneingangsschnittstelle an die erfindungsgemäße Datenverarbeitungsvorrichtung weitergeleitet. Die Dateneingangsschnittstelle kann dabei mittels eines Kabels oder drahtlos ausgeführt sein. Drahtlose Varianten sind dem Fachmann in einer Vielfalt bekannt. Beispielhaft genannt seien Infrarotschnittstellen, Funkschnittstellen, Bluetooth-Schnittstellen etc.

Nach einer erfolgten Messung mit einem konventionellen Blutzuckermessgerät wird das Ergebnis der Messung an die erfindungsgemäße Datenverarbeitungsvorrichtung übertragen. Dieses gibt das Ergebnis der Messung bzw. den zugehörigen Basisdatensatz akustisch aus. Der Basisdatensatz umfasst die Uhrzeit und das Datum der Messung und den ermittelten Blutzuckerwert. Alternativ können mehrere Messungen vorgenommen und in dem Blutzuckermessgerät zwischengespeichert werden, bevor sie an die Datenverarbeitungsvorrichtung übermittelt werden. Nach der Übertragung eines oder mehrerer Basisdatensätze werden diese in der Datenverarbeitungsvorrichtung automatisch gespeichert.

Erfindungsgemäß kann ein Nutzer weitere physiologische Parameter, wie Insulinmengen, Broteinheiten usw. manuell über die Eingabeeinheit (bspw. eine numerische Tastatur der Eingabeeinheit) eingeben und zu einem bspw. automatisch übermittelten Basisdatensatz hinzuspeichern. Die Größe der Speichereinheit ist so gewählt, dass die Datenverarbeitungsvorrichtung eine ausreichende Anzahl derartiger Datensätze speichern kann, um ein ausführliches Tagebuch anlegen zu können. Bspw. kann die Größe der Speichereinheit derart gewählt sein, dass mehrere Tausend derartiger Datensätze abgespeichert werden können. Mit den heutzutage verfügbaren Technologien dürfte es unproblematisch sein, die Speichereinheit so auszulegen, dass mehrere 10.000 derartiger Datensätze abgespeichert werden.

Mittels der Eingabeeinheit kann ein Nutzer der Datenverarbeitungsvorrichtung aus der Vielzahl von abgespeicherten Datensätzen gewünschte Datensätze selektieren. Erfindungsgemäß kann die Selektion derart erfolgen, dass der Nutzer über die Eingabeeinheit für jeden Parameter der Basisdatensätze Zielbereiche bzw. Intervallgrenzen eingibt. Die Schnittmenge der eingegebenen Zielbereiche für die unterschiedlichen Parameter ergibt ein Suchcluster, nach dem die Suche durchgeführt wird. Grundsätzlich sind sowohl Und-Verknüpfungen als auch Oder-Verknüpfungen der einzelnen Parameter einer Selektion möglich. So kann ein Nutzer des Geräts bspw. als Zielbereiche für die Parameter Datum und Insulinwert eine Suche nach allen Datensätzen zwischen dem 21. April 2006 und dem 25. September 2006 mit Insulinwerten über 20 IE (Internationalen Einheiten) durchführen.

Die derart ermittelten Datensätze können bspw. akustisch ausgegeben werden. Damit wird einem blinden bzw. sehbehinderten Nutzer der Zugang zu Tagebucheinträgen mittels einer Sprachausgabe von einzelnen selektierten Daten ermöglicht und somit der Zugriff auf eine ausgewählte Datenmenge aus einer großen Anzahl von Daten überhaupt erst ermöglicht. Auch sehende Nutzer können hiervon profitieren. Um die Ausgabe der Daten für den Nutzer zur Markierung und Erkennung der Treffer besser erfassbar zu machen, kann jedem selektierten Datensatz zur Abgrenzung gegen mitselektierte Datensätze (Begleitdatensätze), die vor bzw. nach dem Treffer liegen, ein akustisches Signal vorangestellt werden. Daher umfasst die Erfindung auch ein Sprachausgabegerät mit einer erfindungsgemäßen Datenverarbeitungsvorrichtung.

Die Eingabeeinheit der erfindungsgemäßen Datenverarbeitungsvorrichtung umfasst zum Steuern eines virtuellen Cursors eine Vorwärtstaste und eine Rückwärtstaste. Mittels dieser Tasten kann der blinde Benutzer in den selektierten Datensätzen navigieren. Mittels der Vorwärts- bzw. Rückwärtstaste der Eingabeeinheit kann der Nutzer zu einem durch das vorangestellte akustische Signal ausgewiesenen selektierten Datensatz vorangegangene bzw. nachfolgende Datensätze abrufen. Diesen sogenannten Begleitdatensätzen ist kein akustisches Signal vorangestellt. Mittels dieses Auswahlverfahrens, das eine zweite Selektion im Sinne der vorliegenden Erfindung darstellt, kann der Nutzer ausgehend von einem selektierten Datensatz die historische bzw. chronologische Entwicklung der Messwerte direkt vor bzw. direkt nach dem betreffenden selektierten Datensatz nachvollziehen. Um dem Nutzer die unterschiedlichen Ebenen der Sprachausgabe zu verdeutlichen, werden diesen Begleitdatensätzen keine akustischen Signale vorangestellt. Alternativ kann diesen Begleitdatensätzen ein anderes akustisches Signal als dem selektierten Datensatz vorangestellt werden. Auf diese Art und Weise hat der blinde Nutzer der erfindungsgemäßen Datenverarbeitungsvorrichtung die Möglichkeit, ähnliche physiologische Zustände wie den aktuellen in der Vergangenheit zu identifizieren und die damals getroffenen Maßnahmen wieder einzuleiten oder zu optimieren (die damals getroffenen Maßnahmen sind mittels der zusätzlich abgespeicherten physiologischen Parameter nachvollziehbar). In weiterer alternativer Ausgestaltung wird ein zweites akustisches Signal verwendet, um unterschiedliche Zeitintervalle zu markieren, in denen der virtuelle Cursor bewegt wird.

Die Erfindung gestattet dem blinden Benutzer in einem aus einer Vielzahl von Datensätzen bestehenden elektronischen Tagebuch effektiv zu navigieren. Jeder Datensatz besteht aus mehreren Datensatzfeldern. Im Falle eines Blutzuckermessgerätes kann ein sog. Basisdatensatz die Datensatzfelder Datum, Uhrzeit und Blutzuckerwert umfassen. Zusätzlich zu den Datensatzfeldern des. Basisdatensatzes können noch zusätzliche Datensatzfelder vorgesehen sein, die von dem Benutzer frei belegbar sind. Im Falle eines Blutzuckermessgerätes kann es sich bei zusätzlichen Datensatzfeldern insbesondere um physiologische Parameter, wie bspw. Insulinmengen und Broteinheiten handeln. Die Erfindung stellt dem blinden Benutzer mehrere Möglichkeiten dar, in dem umfangreichen Datensatzbestand zu navigieren. Eine erste Möglichkeit besteht darin, die vorhandenen Datensätze in chronologischer Reihenfolge abzurufen. Dies kann bspw. durch Betätigen der Vorwärts- bzw. Rückwärtstaste erfolgen. Bspw. kann durch Betätigen der Rückwärtstaste der jüngste Datensatz im Gesamtdatenbestand selektiert und akustisch ausgegeben werden. Durch nochmaliges Betätigen der Rückwärtstaste wird dann der chronologisch direkt davor liegende Datensatz selektiert und akustisch ausgegeben usw. Durch Betätigen der Vorwärtstaste kann im Gegenzug der älteste Datensatz im Datensatzbestand selektiert und akustisch ausgegeben werden, durch nochmaliges Betätigen der Vorwärtstaste der zweitälteste Datensatz usw. Durch längeres Betätigen der zugeordneten Taste kann eine Sprungfunktion aktiviert werden, die bewirkt, dass als nächster Datensatz derjenige Datensatz ausgegeben wird, der in einem voreingestellten oder einstellbaren Abstand zum als letztes ausgegebenen Datensatz selektiert und akustisch ausgegeben wird. Bei dem zeitlichen Abstand bzw. Sprung kann es sich bspw. um einen Tag, zwei Tage, eine Woche, 14 Tage, einen Monat usw. handeln. Unterschiedliche zeitliche Abstände/Sprünge können durch unterschiedliche akustische Signale markiert sein.

Eine zweite Möglichkeit des Navigierens besteht darin, nach einem physiologischen Parameter/Zustand zu suchen. Physiologische Parameter können von einem Nutzer frei in dazu vorgesehene definierbare Datensatzfelder oder vorgegebene Felder eingegeben werden. Um die Benutzbarkeit der Datenverarbeitungsvorrichtung für einen blinden Benutzer möglichst einfach zu gestalten, verfügt die Datenverarbeitungsvorrichtung lediglich über eine Zifferntastatur und keine Buchstabentastatur. Daher definiert der Benutzer bestimmte physiologische Parameter, wie bspw. Insulinmengen, oder andere Stichworte, wie bspw. "Sport" oder "nach dem Mittagessen" durch entsprechende Kennziffern. Der Vorgang des Selektierens gestaltet sich dann so, dass der Benutzer zunächst mit einer zugeordneten Taste (bspw. die Vorwärts-/Rückwärtstaste) die Selektionsfunktion startet und anschließend interaktiv nach entsprechender Aufforderung durch die Datenverarbeitungsvorrichtung ein Selektionskriterium eingibt. Bei dem Selektionskriterium handelt es sich um einen Parameter wie bspw. "Sport", der als entsprechende Kennziffer, mit der er definiert ist, eingegeben wird. Nach Eingabe der Kennziffer und ggf. Betätigen einer entsprechenden Bestätigungstaste wird die Selektion durch die Recheneinheit der Datenverarbeitungsvorrichtung vorgenommen und alle Datensätze mit einem Datensatzfeldeintrag "Sport" werden selektiert. Durch Betätigen einer Abruftaste bzw. der Vorwärts- bzw. Rückwärtstaste kann der Nutzer die einzelnen selektierten Datensätze sukzessiv akustisch ausgeben lassen.

Den Datensätzen wird bei der akustischen Ausgabe bspw. ein bestimmtes akustisches Signal vorangestellt. Mit einer entsprechenden zugeordneten Taste, bspw. eine Cursortaste oder einer Links-/Rechtstaste oder Vorwärts-/Rückwärtstaste können zu jedem selektierten Datensatz die jeweils chronologisch benachbarten Datensätze abgerufen werden. Diesen sogenannten Begleitdatensätzen ist kein akustisches Signal vorangestellt. Alternativ kann diesen sog. Begleitdatensätzen ein zweites akustisches Signal vorangestellt sein, das sich von dem ersten akustischen Signal unterscheidet. Diese Begleitdatensätze sind nützlich, um die Historie der selektierten Datensätze darzustellen. Der Nutzer hat damit die Möglichkeit, ähnlich physiologische Zustände wie den aktuellen in der Vergangenheit zu identifizieren und die damals getroffenen Maßnahmen wieder einzuleiten oder zu optimieren.

Schließlich besteht als weitere Möglichkeit der Navigation die Eingabe von Zielbereichen, d.h. Intervallgrenzen für unterschiedliche Parameter. Dabei kann diese Intervallselektion durch Eingabe von Intervallgrenzen für ein Datensatzfeld oder durch Eingabe von Intervallgrenzen für mehrere Datensatzfelder durchgeführt werden. Bspw. kann als Zielbereich ein Datumsintervall eingegeben werden, woraufhin alle Datensätze selektiert werden, die innerhalb des eingegebenen Datumsintervalls liegen. Ergänzend kann noch ein Intervall für bspw. Insulinwerte eingegeben werden, was zur Folge hat, dass alle Datensätze innerhalb eines bestimmten zeitlichen Bereichs mit bestimmten Insulinwerten selektiert werden. Dies ermöglicht eine beliebige Kombination aller Zielbereiche aller Parameter und somit eine individuelle und umfassende Navigation des blinden Benutzers durch den Gesamtdatenbestand.

Erfindungsgemäß ist vorgesehen, dass die Datenverarbeitungsvorrichtung dem Nutzer in Reaktion auf die erste Selektion Auswahlmöglichkeiten für eine medizinisch sinnvolle zweite Selektion bereitstellt. Die Auswahlmöglichkeiten für eine zweite Selektion können eine Berechnung eines Durchschnittswerts aus den durch die erste Selektion ermittelten Datensätzen, eine innerhalb eines bestimmten Zeitraums verabreichte Insulinmenge, Extremwerte des Glucosewerts innerhalb der durch die erste Selektion ermittelten Datensätze, eine Anzahl der nach oder vor den durch die erste Selektion bestimmten Datensätzen innerhalb eines bestimmten Zeitraums aufgetretenen Hypo- oder Hyperglykämien oder bestimmte, mit den durch die erste Selektion bestimmten Datensätzen verknüpfte Ereignisse umfassen.

Dabei können die Hyper- und Hypoglykämien individuell und abhängig von bestimmten zusätzlichen, mit den Glucosemesswerten verknüpften Angaben, wie etwa Mittagessen, definiert sein, etwa < 60 mg/dl für eine Hypoglykämie und >180 mg/dl für eine Hyperglykämie.

Es kann vorgesehen sein, dass eine akustische Ausgabe grundsätzlich aller Datensatzfelder eines Datensatzes erfolgt, und nicht nur derjenigen Datensatzfelder, nach denen selektiert wurde. Auf diese Weise ist der Nutzer in der Lage, die Informationen im Kontext zu bewerten.

Des weiteren kann vorgesehen sein, dass die erste und die zweite Selektion logisch mit einer Und-Verknüpfung miteinander verbunden sind.

Zusätzlich können dem Nutzer auch weitere Funktionen als Auswahlmöglichkeiten angeboten werden, bspw. ein Export der selektierten Datensätze über USB (Universal Serial Bus), Bluetooth oder IR (Infrarot-Schnittstelle), ein Verschieben der Datensätze im Tagebuch, eine akustische Ausgabe nur derjenigen Datensatzfelder, nach denen selektiert wurde, die Verknüpfung der ersten und der zweiten Selektion über eine Oder-Verknüpfung, weitere individuelle logische Verknüpfungen einzelner Datensatzfelder oder eine Korrektur/Änderung bereits gespeicherter Datensätze.

Es kann selbstverständlich vorgesehen sein, dass nach der zweiten Selektion weitere Selektionen stattfinden und dem Nutzer auch hierfür Auswahlmöglichkeiten bereitgestellt werden.

Die beschriebenen Möglichkeiten zur Eingabe mittels der Eingabeeinheit und zum Navigieren innerhalb der selektierten Datensätze finden selbstverständlich auch auf die zweite und eventuelle weitere Selektionen Anwendung.

Die Erfindung umfasst auch ein Computerprogramm mit Programmcode, der dazu geeignet ist, ein Verfahren gemäß der Erfindung durchzuführen, wenn das Computerprogramm auf einer geeigneten Recheneinrichtung, bspw. einer Datenverarbeitungsvorrichtung mit einer Recheneinheit, abläuft. Das Computerprogramm kann als sogenannte Embedded Software auf einer Datenverarbeitungsvorrichtung gespeichert sein, es kann aber auch über eine geeignete Schnittstelle von einem geeigneten Medium auf die Datenverarbeitungsvorrichtung überspielt werden.

Weitere Vorteile und Ausgestaltungen der Erfindung ergeben sich aus der Beschreibung und der beiliegenden Zeichnung.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist anhand eines Ausführungsbeispieles in der Zeichnung schematisch dargestellt und wird im folgenden unter Bezugnahme auf die Zeichnung ausführlich beschrieben.
Figur 1 zeigt in schematischer perspektivischer Ansicht ein Ausführungsbeispiel einer erfindungsgemäßen Datenverarbeitungsvorrichtung.
Figur 2 zeigt den Aufbau der Datenverarbeitungsvorrichtung der Figur 1 darstellendes Blockdiagramm.

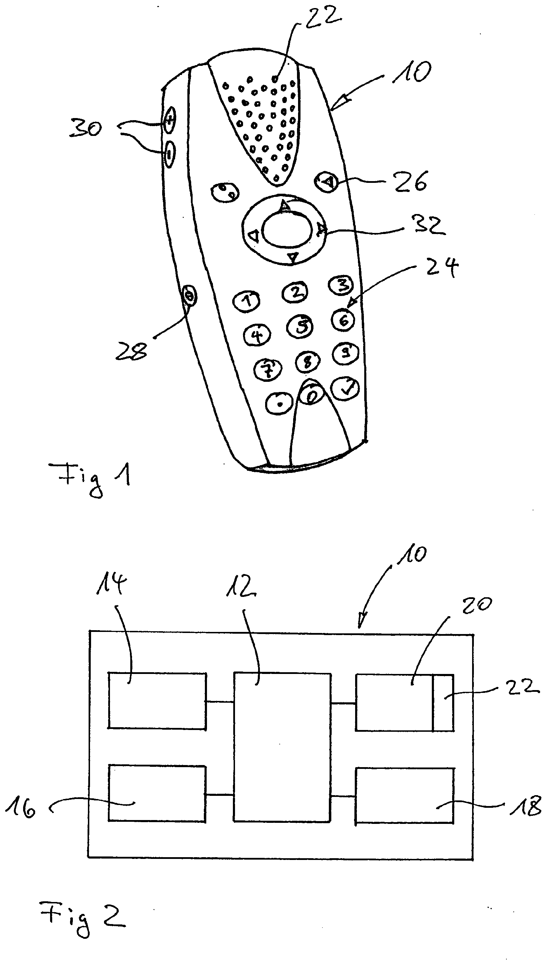

Eine als Sprachausgabegerät ausgebildete erfindungsgemäße Datenverarbeitungsvorrichtung 10 ist in Figur 1 in perspektivischer Darstellung und in schematischer Blockdarstellung in Figur 2 gezeigt.

Die Datenverarbeitungsvorrichtung 10 umfasst eine Recheneinheit 12, eine erste Speichereinheit 14, eine zweite Speichereinheit 16, eine Eingabeeinheit 18 sowie eine Ausgabeeinheit 20 mit einer akustischen Datenausgangsschnittstelle 22. Die akustische Datenausgangsschnittstelle 22 ist bspw. ein Lautsprecher (vgl. Figur 1) und/oder eine Kopf- bzw. Ohrhörerbuchse.

Des weiteren umfasst die Datenverarbeitungsvorrichtung 10 eine Mehrzahl von (Bestandteil der Eingabeeinheit 18 bildenden) Tasten, anhand derer eine Bedienperson die Datenverarbeitungsvorrichtung 10 bedienen und benutzen kann. Bei den Tasten handelt es sich insbesondere um eine numerische Tastatur 24 (im gezeigten Ausführungsbeispiel wie bei einem Telefon angeordnet) sowie um Steuerungstasten 32 (Pfeiltasten), Eingabebestätigungstaste 26, Ein/Aus-Taste 28, +/-Tasten 30 zur Lautstärkeeinstellung u.a. Es versteht sich, dass die Ausgestaltung der Eingabeeinheit und insbesondere Art und Weise und Umfang der Tastatur nicht auf die dargestellte Ausführungsform beschränkt ist und der Fachmann auch anders gestaltete Tastaturanordnungen in Betracht zieht.

Die Eingabeeinheit umfasst des weiteren eine oder mehrere (nicht dargestellte) insb. serielle Schnittstellen zur Dateneingabe, wie bspw. eine Infrarotschnittstelle, eine serielle Datenschnittstelle und/oder eine USB-Schnittstelle. Alternativ kann bspw. auch eine Bluetooth-Schnittstelle o.dgl. vorgesehen sein.

In der ersten Speichereinheit 14 sind bspw. Tondateien abgespeichert, aus denen sich die Sprachausgabe der Datenverarbeitungsvorrichtung erzeugen lässt (wie dies in der parallelen europäischen Patentanmeldung Nr. 06 025 798.7 der Anmelderin beschrieben ist).

In der zweiten Speichereinheit 16 sind einen Datensatzbestand bildende Datensätze abgespeichert, die zumindest teilweise auf messtechnisch erhobenen Daten beruhen. Die Datensätze können im Beispiel einer medizinischen Datenverarbeitungsvorrichtung von einem Blutzuckermessgerät oder einem anderen medizinischen Messgerät stammen und mittels einer geeigneten Schnittstelle (wie voranstehend beschrieben) auf die erfindungsgemäße Datenverarbeitungsvorrichtung übertragen und dort in der zweiten Speichereinheit 16 abgespeichert werden. Ein derart von einem externen Gerät messtechnisch erhobener und übertragener Datensatz wird im Rahmen dieser Anmeldung als Basisdatensatz bezeichnet. Ein Basisdatensatz umfasst mindestens zwei Datensatzfelder. Bspw. umfasst ein Basisdatensatz im Bereich der Blutzuckermessung drei Datensatzfelder, das Datum und die Uhrzeit der Messwerterhebung sowie den gemessenen Blutzuckerwert.

Ein Basisdatensatz kann sofort nach Übertragung automatisch in der zweiten Speichereinheit 16 gespeichert und über die akustische Datenausgangsschnittstelle (z.B. Lautsprecher 22) ausgegeben werden.

Darüber hinaus bietet die Datenverarbeitungsvorrichtung gemäß der Erfindung dem Benutzer die Möglichkeit, weitere Datensatzfelder selbst zu definieren und für ihn wichtige bzw. relevante Werte, Daten oder andere Angaben einzugeben. Bei diesen Werten, Daten oder anderen Angaben kann es sich bspw. um weitere physiologische Parameter, wie Insulinmengen, Broteinheiten usw., handeln. Des weiteren können bspw. Angaben wie "Sport", "Mittagessen" o.dgl. eingegeben werden. Dadurch wird dem blinden Benutzer die Möglichkeit eröffnet, ein Tagebuch über die Entwicklung seines Gesundheitszustandes zu führen.

Der Datensatzbestand eines derartigen medizinischen Tagebuchs wird nach einer Zeit der Benutzung eine hohe Zahl von Datensätzen umfassen. Um einem blinden Benutzer zu ermöglichen, in dem Datensatzbestand zu navigieren und ihm die Inhalte zugänglich zu machen (also ihm die Auswertung seines elektronischen medizinischen Tagebuchs zu ermöglichen), wird erfindungsgemäß eine interaktive zweistufige Selektion von Daten angeboten, die an die Bedürfnisse einer blinden Person angepaßt ist. Die Selektion erfolgt dabei bspw. nach Inhalten auszuwählender Datensatzfelder.

Der blinde Benutzer kann bspw. erfindungsgemäß durch Betätigen einer zugeordneten Taste, bspw. der Vorwärts/Rückwärtstaste (Pfeiltasten 32), Datensätze in chronologischer Selektion akustisch abrufen. Durch einmaliges Betätigen (bspw. kurzes Drücken) der Rückwärtstaste (bspw. Pfeil nach links) wird der Datensatz mit dem jüngsten Datum und Uhrzeit selektiert und akustisch ausgegeben. Die akustische Ausgabe selbst kann dabei nach einem der dem Fachmann bekannten Sprachausgabeverfahren oder nach dem Verfahren der Anmelderin, wie es in der parallelen Anmeldung europäischen Patentanmeldung Nr. 06 025 798.7 beschrieben ist, erfolgen.

Durch erneutes einmaliges Betätigen der Rückwärtstaste wird der nächstjüngste Datensatz selektiert und akustisch ausgegeben usw. Bei der akustischen Ausgabe kann jedem Datensatz erfindungsgemäß ein (erstes) akustisches Signal vorangestellt werden, bspw. ein "Ping". Wünscht der Benutzer eine Ausgabe beginnend mit dem ältesten Datensatz, so kann er dies analog durch Betätigen der entsprechenden zugeordneten Taste, bspw. der Vorwärtstaste, erreichen. Durch beliebiges Betätigen der beiden zugeordneten Tasten kann so in beliebiger chronologischer Richtung durch den Datensatzbestand hin- und hernavigiert werden.

Der Benutzer kann auch eine sogenannte Sprungfunktion aktivieren, mit der er Datensätze mit voreingestellten oder auswählbaren zeitlichen Abständen selektiert. Der zeitliche Abstand kann bspw. 7 Tage, 14 Tage oder einen Monat betragen. Die Aktivierung der Sprungfunktion kann durch längeres Betätigen der zugeordneten Taste erfolgen. Selbstverständlich kann es auch andere Möglichkeiten einer Aktivierung der Sprungfunktion geben, wie bspw. das Betätigen einer Funktionstaste, mit der die Funktion der entsprechenden zugeordneten Taste (bspw. Rückwärts/Vorwärtstaste) auf einen Sprungfunktionsmodus umgestellt wird.

Um die Ausgabe von Datensätzen bei aktivierter Sprungfunktion von den Datensätzen bei nicht aktivierter Sprungfunktion zu unterscheiden, kann erfindungsgemäß den Datensätzen bei aktivierter Sprungfunktion ein akustisches Signal vorangestellt werden, das sich von dem ersten akustischen Signal unterscheidet, bspw. in der Tonlage. Da blinde Benutzer in der Regel ein sehr sensitiv entwickeltes Gehör haben, können sie auf diese Weise die unterschiedlichen Datensätze voneinander unterscheiden. Alternativ können unterschiedliche Sprungfunktionen durch unterschiedliche akustische Signale voneinander unterschieden werden.

Selbstverständlich können auch andere Kombinationen von akustischen Signalen gewählt werden, um eine Unterscheidung zuzulassen. So kann bspw. die Ausgabe im Grundmodus bei kurz gedrückter zugeordneter Taste ohne vorangestelltes akustisches Signal (sozusagen ein nicht hörbares Signal) erfolgen, während bei aktivierter Sprungfunktion ein Signal vorangestellt wird. Des weiteren ist es für den Fachmann auf der Grundlage der vorliegenden technischen Lehre ohne weiteres möglich, eine Datenverarbeitungsvorrichtung mit mehreren Stufen von Sprungfunktionen zu konzipieren.

Eine weitere Selektionsmöglichkeit ist die Eingabe von Zielbereichen. Darunter wird im Rahmen der vorliegenden Erfindung das Eingeben von Intervallgrenzen, also eine Intervallselektion, verstanden. Bei der Intervallselektion kann für jeden Parameter (also jedes Datensatzfeld) ein Intervall bzw. ein Zielbereich eingegeben werden, nach dem dann eine Selektion der Datensätze erfolgt. Außerdem ist eine Kombination von mehreren Zielbereicheingaben möglich, deren Ergebnis dann eine Schnittmenge der für jeden Parameterzielbereich selektierten Datensätze ist.

Als Beispiel wird eine Selektion nach allen Datensätzen durchgeführt, die zwischen dem 21. Juni 2006 und dem 04. Oktober 2006 liegen und Insulinwerte über 20 i.E. enthalten. Dies würde bedeuten, dass der Benutzer als Intervallgrenzen den 21. Juni 2006 und den 04. Oktober 2006 für das Datensatzfeld "Datum" eingibt und als Intervallgrenzen 20 und Unendlich für das Datensatzfeld "Insulinwert" eingibt. Die Eingabe erfolgt interaktiv, gesteuert durch eine (hier nicht näher beschriebene) Sprachsteuerung der Datenverarbeitungsvorrichtung. In anderen Beispielen kann das Intervall auf einen einzigen Wert beschränkt sein; so könnten bspw. durch Eingabe von 12:00 Uhr für die untere und die obere Intervallgrenze für das Datensatzfeld "Uhrzeit" alle Datensätze ermittelt werden, die um genau 12:00 Uhr mittags erhoben wurden, wenn der Benutzer seine Insulinwerte um diese Uhrzeit selektieren und akustisch ausgeben lassen will. Wird nur eine Untergrenze (bspw. 12:00 Uhr) eingegeben, so werden alle Werte darüber (also größer als 12:00 Uhr) ermittelt, wird der Wert entsprechend nur als Obergrenze eingegeben, so werden alle Werte darunter (kleiner 12:00 Uhr) ermittelt.

Die akustische Ausgabe erfolgt bspw. automatisch nach durchgeführter Selektion oder nach Betätigen einer entsprechenden Taste (Abspieltaste) durch den Benutzer.

Die akustische Ausgabe eines jeden Treffers/Datensatzes erfolgt bspw. mit einem vorangestellten (ersten) akustischen Signal.

Die Erfindung ermöglicht dem Benutzer auch, als medizinisch sinnvolle zweite (oder weitere) Selektion sogenannte benachbarte Datensätze zu einem selektieren Datensatz anzuhören. Unter einem benachbarten Datensatz ist im Rahmen der vorliegenden Erfindung ein Datensatz zu verstehen, der nicht notwendigerweise Bestandteil der Schnittmenge der Datensatzselektion ist, zeitlich jedoch direkt vor oder nach einem selektierten Datensatz liegt. Sind z.B. nach dem voranstehenden Beispiel alle Datensätze zwischen dem 21. Juni 2006 und dem 04. Oktober 2006 mit einem Insulinwert über 20 i.E. selektiert worden, so wird der erste ermittelte Datensatz (bspw. der älteste oder der jüngste Datensatz) akustisch ausgegeben. Nach der Ausgabe des Datensatzes kann der Benutzer entscheiden, ob er einen benachbarten Datensatz, der zeitlich vor oder nach dem ausgegebenen Datensatz liegt, akustisch ausgeben lassen will. Dazu betätigt er eine entsprechende zugeordnete Taste (bspw. die Seitwärtstasten Pfeil links bzw. Pfeil rechts). Zu jedem selektierten Datensatz können einer oder mehrere benachbarte Datensätze in jeder zeitlichen Richtung ausgegeben werden, bspw. jeweils die drei nächstliegenden Datensätze in zeitlicher Richtung vor und nach dem selektierten Datensatz. Diese Datensätze können auch als Begleitdatensätze des selektierten Datensatzes bezeichnet werden. Den Begleitdatensätzen kann bei der Ausgabe ein akustisches Signal vorangestellt werden, das sich von dem dem selektierten Datensatz vorangestellten ersten akustischen Signal unterscheidet.

Die Intervallselektion gemäß der Erfindung bietet dem blinden Benutzer die Möglichkeit, ähnliche physiologische Zustände wie den aktuellen in der Vergangenheit zu identifizieren und ggf. die damals getroffenen Maßnahmen (die in den individuell definierten Datensatzfeldern gespeichert sind) anzuhören und diese dann erneut einzuleiten (oder zu optimieren).

Darüber hinaus bietet die Erfindung dem Benutzer als weitere Selektionsmöglichkeit die Selektion nach Inhalten von individuell definierten Datensatzfeldern. Diese Möglichkeit der Selektion kann auch als Stichwortselektion oder, im Falle einer medizinischen Datenverarbeitungsvorrichtung, Selektion nach physiologischen Parametern/Zuständen genannt werden.

Wie bereits voranstehend beschrieben, kann der blinde Benutzer Datensatzfelder für spezielle und für ihn wichtige physiologische Zustände definieren und sogenannte Basisdatensätze dann mit entsprechenden Eingaben in diese Datensatzfelder ergänzen. Dies können neben den bereits genannten Insulinwerten auch Aussagen sein wie "Sport", "Stress" usw., die den Gesundheitszustand beeinflussen oder prägen können. Für das Auffinden dieser Parameter wird die Stichwortselektion angewendet. Mit Hilfe der Stichwortselektion können bestimmte Begriffe bzw. Aussagen in den zusätzlichen, individuell definierten Datensatzfeldern gesucht werden. Dies kann bspw. so erfolgen, dass nach Aufrufen der Stichwortselektion (bspw. interaktiv durch ein akustisches Menü oder durch Betätigen einer zugeordneten Taste oder Tastenkombination) der Benutzer eine Kennziffer eingibt, die für den gesuchten Begriff vorgegeben oder individuell definiert wurde (oder die Selektion kann erfolgen, indem der virtuelle Cursor auf dem zu suchenden Parameter steht und die Vorwärts- oder Rückwärtstaste betätigt wird). Die dadurch ausgelöste Selektion kann automatisch oder durch Betätigen einer Taste, bspw. der Vorwärts/Rückwärtstaste, sukzessiv akustisch ausgegeben werden. Ist bspw. die Kennziffer "3" für den Begriff "Sport" definiert, so werden nach Eingabe der Ziffer "3" alle Datensätze selektiert, die in einem Datensatzfeld den Begriff "Sport" (besser gesagt: die für den Begriff "Sport" stehende Ziffer "3") enthalten. Durch Betätigen bspw. der Rückwärtstaste wird dem Benutzer dann derjenige Datensatz aus den selektierten Datensätzen akustisch ausgegeben, der das jüngste Datum hat. Der Benutzer kann dann nach Ausgabe dieses Datensatzes durch erneutes Betätigen den nächstjüngsten Datensatz abrufen usw. Selbstverständlich kann auch für die Ausgabe des Ergebnisses einer Stichwortselektion ein Sprungfunktionsmodus vorgesehen sein, und selbstverständlich kann auch für die Ausgabe des Ergebnisses einer Stichwortselektion die Möglichkeit des Abrufens von Begleitdatensätzen (vgl. oben) vorgesehen sein.

Im Anschluss an eine erste Selektion wird dem Nutzer dann erfindungsgemäß eine zweite, medizinisch sinnvolle Selektion zur Einschränkung oder Ergänzung des Ergebnisses der ersten Selektion angeboten. Bei dieser zweiten Selektion ist vorgesehen, dass dem Nutzer basierend auf der ersten Selektion Auswahlmöglichkeiten bereitgestellt werden. Die Auswahlmöglichkeiten für eine zweite Selektion können eine Berechnung eines Durchschnittswerts aus den durch die erste Selektion ermittelten Datensätzen, eine innerhalb eines bestimmten Zeitraums verabreichte Insulinmenge, Extremwerte des Glucosewerts innerhalb der durch die erste Selektion ermittelten Datensätze, eine Anzahl der nach oder vor den durch die erste Selektion bestimmten Datensätzen innerhalb eines bestimmten Zeitraums aufgetretenen Hypo- oder Hyperglykämien oder bestimmte, mit den durch die erste Selektion bestimmten Datensätzen verknüpfte Ereignisse umfassen.

So kann bspw. vorgesehen sein, dass einem Nutzer bei einer ersten Selektion anhand eines Datensatzfeldes mit der zusätzlichen Angabe "Sport" angeboten wird, sämtliche Datensätze akustisch auszugeben, die innerhalb von bspw. 12 Stunden nach mit "Sport" verknüpften Messungen hyper- oder hypoglykämische Zustände darstellen. Der Nutzer kann dann etwa über die ebenfalls in diesen Datensätzen gespeicherten Informationen zu verabreichten Insulinmengen sukzessive Verbesserungen in seinem Therapieverhalten herbeiführen, um hyper- oder hypoglykämische Zustände zu vermeiden.

Unter dem Begriff "medizinisch sinnvoll" ist somit im Rahmen dieser Anmeldung jede Vorgehensweise zu verstehen, die darauf abzielt, dem Benutzer ein möglichst akkurates und umfassendes Bild über seinen Zustand, also z.B. seinen Blutzuckerzustand, zu vermitteln. Insbesondere soll der Benutzer aufgrund der gezielten Vorgabe von Auswahlmöglichkeiten in die Lage versetzt werden, hyper- oder hypoglykämische Zustände zu vermeiden.

Im Zusammenhang mit der zweiten Selektion bieten sich dem Nutzer selbstverständlich ebenfalls diejenigen Möglichkeiten zur Eingabe und zur Navigation, wie sie bereits voranstehend beschrieben wurden.

Es kann somit sowohl nach Ereignissen wie bspw. Sport, Essen, Stress, usw. als auch nach bestimmten Messbereichen (Intervallen) gesucht werden. Bei einer chronologischen Auflistung der selektierten Daten kann in zwei verschiedenen Modi durch die Daten hindurchgeführt werden; zum einen kann in Einzelschritten von einem zum nächsten Treffer mit der Cursortaste gehüpft werden, zum anderen kann bei längerem Halten der Cursortaste in zeitlichen Abständen (bspw. wochenweise) durch die Ereignisse gehüpft werden, wobei in letzterem Fall ein zweiter Signalton (zweites akustisches Signal) vorangestellt wird.

Mit der vorliegenden Erfindung hat ein blinder Benutzer zum ersten Mal die Möglichkeit, eigenständig, eigenverantwortlich und ohne fremde Hilfe ein medizinisches Tagebuch zu führen. Er hat die Möglichkeit, seinen Blutzucker einzustellen und historische Daten zu "sichten", d.h. durch diesen Bestand an historischen Daten derart zu navigieren, dass er bzw. sie als blinde Person auf die Dateninhalte ohne fremde Hilfe zugreifen kann. Dadurch kann die blinde Person Fehler bei der Einstellung des Blutzuckers vermeiden und so weiteren gesundheitlichen Komplikationen vorbeugen. Die Erfindung gestattet somit ein zeitnahes Auffinden von Daten, die früher nur mühsam zugänglich waren. Das zeitnahe Auffinden ist von großer Bedeutung, da in kritischen Situationen der Körper durch Gaben von entsprechenden Insulinmengen schnell in ein Gleichgewicht gebracht werden muss.

Selbstverständlich können die Daten über eine entsprechende Schnittstelle vollständig oder nach Selektion in einen Computer mit einem geeigneten Tabellenprogramm exportiert werden, damit sie dort bspw. von einem behandelnden Arzt gesichtet und ausgewertet werden können (Erkennung von physiologischen Reaktionen des Nutzers, Entwicklung geeigneter Therapiemaßnahmen).

## Patentansprüche

1. Datenverarbeitungsvorrichtung zur Verarbeitung von Messwerten von einem medizinischen Messgerät, mit einer Eingabeeinheit (18), einer Dateneingangsschnittstelle zur Kommunikation mit einem zugeordneten Messgerät, einer Speichereinheit (14, 16) zum Speichern von über die Eingabeeinheit (18) eingegebenen und/oder über die Dateneingangsschnittstelle empfangenen Datensätzen und einer Recheneinheit (12), wobei die Datenverarbeitungsvorrichtung (10) derart ausgestaltet und eingerichtet ist, dass ein Nutzer mittels der Eingabeeinheit (18) eine erste Selektion von Datensätzen aus den in der Speichereinheit (14, 16) gespeicherten Datensätzen auslösen kann,
**dadurch gekennzeichnet, dass** die Datenverarbeitungsvorrichtung (10) des weiteren eine akustische Datenausgangsschnittstelle (22) aufweist und derart ausgestaltet und eingerichtet ist, dass eine Ausgabe der selektierten Datensätze automatisch nach durchgeführter Selektion oder nach Betätigen einer entsprechenden Taste durch den Benutzer akustisch über die akustische Datenausgangsschnittstelle (22) erfolgt und dass die akustische Ausgabe derart erfolgt, dass jedem selektierten Datensatz bei der akustischen Ausgabe zur Abgrenzung gegen Begleitdatensätze, die vor bzw. nach dem Treffer liegen, ein erstes akustisches Signal vorausgeht, und wobei den Begleitdatensätzen bei der Ausgabe kein akustisches Signal vorangestellt wird oder ein zweites akustisches Signal vorangestellt wird, das sich von dem ersten akustischen Signal unterscheidet.

2. Datenverarbeitungsvorrichtung nach Anspruch 1, bei dem die akustische Datenausgangsschnittstelle (22) ein Lautsprecher und/oder ein Anschluss für einen Kopf- bzw. Ohrhörer ist.

3. Datenverarbeitungsvorrichtung nach Anspruch 1 oder 2, bei dem die Eingabeeinheit (18) eine Vorwärtstaste und eine Rückwärtstaste (32) zum Steuern eines virtuellen Cursors, eine Auswahltaste (46) oder eine numerische Tastatur (24) umfasst.

4. Datenverarbeitungsvorrichtung nach einem der Ansprüche 1 bis 3, bei dem die Dateneingangsschnittstelle eine drahtlose Schnittstelle zur Kommunikation mit einem Messgerät ist.

5. Datenverarbeitungsvorrichtung nach einem der Ansprüche 1 bis 3, die über die Dateneingangsschnittstelle einstückig mit einem Messgerät ausgebildet ist.

6. Datenverarbeitungsvorrichtung nach einem der Ansprüche 1 bis 5, bei der zumindest die erste Selektion von Datensätzen eine Selektion nach Inhalten eines Datensatzfeldes ist und eine chronologische Selektion, die durch Betätigen einer zugeordneten Taste der Eingabeeinheit (18) erfolgt, oder/und eine Stichwortselektion, die durch Eingabe einer Kennziffer erfolgt, die für ein Selektionskriterium bildendes Stichwort steht, oder/und eine Intervallselektion ist, die durch Eingabe von Intervallgrenzen für ein Datensatzfeld, in dem die Selektion erfolgen soll, erfolgt.

7. Datenverarbeitungsvorrichtung nach einem der Ansprüche 1 bis 6, bei der die Auswahlmöglichkeiten für eine zweite Selektion eine Berechnung eines Durchschnittswerts aus den durch die erste Selektion ermittelten Datensätzen, oder/und eine innerhalb eines bestimmten Zeitraums verabreichte Insulinmenge, oder/und Extremwerte des Glucosewerts innerhalb der durch die erste Selektion ermittelten Datensätze, oder/und eine Anzahl der nach oder vor den durch die erste Selektion bestimmten Datensätzen innerhalb eines bestimmten Zeitraums aufgetretenen Hypo- oder Hyperglykämien oder/und bestimmte, mit den durch die erste Selektion bestimmten Datensätzen verknüpfte Ereignisse oder/und die Ausgabe von Begleitdatensätzen umfassen.

8. Datenverarbeitungsvorrichtung nach einem der Ansprüche 1 bis 7, bei der eine akustische Ausgabe der selektierten Datensätze sowohl nach der ersten als auch der zweiten Selektion erfolgt und jedem selektierten Datensatz bei der akustischen Ausgabe ein akustisches Signal vorausgeht.

9. Datenverarbeitungsvorrichtung nach einem der Ansprüche 1 bis 8, wobei der Nutzer mittels der Eingabeeinheit (18) ein Blättern durch die selektierten Datensätze vornehmen kann.

10. Datenverarbeitungsvorrichtung nach einem der Ansprüche 1 bis 9, wobei eine Sprungfunktion vorgesehen ist, mit der Datensätze mit zeitlichen Abständen selektiert werden.

11. Datenverarbeitungsvorrichtung nach Anspruch 10, wobei den Datensätzen bei aktivierter Sprungfunktion ein akustisches Signal vorangestellt wird, das sich von dem ersten akustischen Signal unterscheidet.

12. Datenverarbeitungsvorrichtung nach einem der Ansprüche 1 bis 11, wobei die Datensätze jeweils eine Mehrzahl von Datenfeldern aufweisen, von denen mindestens ein Datenfeld einen Glucosemesswert beinhaltet, und wobei die Datenverarbeitungsvorrichtung (10) derart ausgestaltet und eingerichtet ist, dass die Datenverarbeitungsvorrichtung (10) dem Nutzer in Reaktion auf die erste Selektion Auswahlmöglichkeiten für eine medizinisch sinnvolle zweite Selektion von Datensätzen aus den durch die erste Selektion ermittelten Datensätzen bereitstellt.

13. Verfahren zum Betrieb einer Datenverarbeitungsvorrichtung zur Verarbeitung von Messwerten von einem Messgerät, wonach eine erste Selektion aus in der Datenverarbeitungsvorrichtung (10) abgespeicherten Datensätzen erfolgt und eine Ausgabe der selektiven Datensätze akustisch über eine akustische Datenausgangsschnittstelle (22) derart erfolgt, dass eine Ausgabe der selektierten Datensätze automatisch nach durchgeführter Selektion oder nach Betätigen einer entsprechenden Taste durch den Benutzer akustisch über die akustische Datenausgangsschnittstelle (22) erfolgt und dass die akustische Ausgabe derart erfolgt, dass jedem selektierten Datensatz bei der akustischen Ausgabe zur Abgrenzung gegen Begleitdatensätze, die vor bzw. nach dem Treffer liegen, ein erstes akustisches Signal vorangestellt wird, und den Begleitdatensätzen bei der Ausgabe kein akustisches Signal vorangestellt wird oder ein zweites akustisches Signal vorangestellt wird, das sich von dem ersten akustischen Signal unterscheidet.

14. Verfahren nach Anspruch 13, bei dem die akustische Datenausgangsschnittstelle (22) ein Lautsprecher und/oder ein Anschluss für einen Kopf- bzw. Ohrhörer ist.

15. Verfahren nach einem der Ansprüche 13 bis 14, bei dem zumindest die erste Selektion von Datensätzen nach Inhalten eines Datensatzfeldes erfolgt und bei dem die zumindest die erste Selektion eine chronologische Selektion ist, die durch Betätigen einer zugeordneten Taste der Eingabeschnittstelle erfolgt, oder/und eine Stichwortselektion, die durch Eingabe einer Kennziffer erfolgt, die für ein Selektionskriterium bildendes Stichwort steht, oder/und eine Intervallselektion ist, die durch Eingabe von Intervallgrenzen für ein Datensatzfeld, in dem die Selektion erfolgen soll, erfolgt.

16. Verfahren nach einem der Ansprüche 13 bis 15, bei dem die Auswahlmöglichkeiten für eine zweite Selektion eine Berechnung eines Durchschnittswerts aus den durch die erste Selektion ermittelten Datensätzen, oder/und eine innerhalb eines bestimmten Zeitraums verabreichte Insulinmenge, oder/und Extremwerte des Glucosewerts innerhalb der durch die erste Selektion ermittelten Datensätze, oder/und eine Anzahl der nach oder vor den durch die erste Selektion bestimmten Datensätzen innerhalb eines bestimmten Zeitraums aufgetretenen Hypo- oder Hyperglykämien oder/und bestimmte, mit den durch die erste Selektion bestimmten Datensätzen verknüpfte Ereignisse oder/und die Ausgabe von Begleitdatensätzen umfassen.

17. Verfahren nach einem der Ansprüche 13 bis 16, durch das ein Blättern durch die selektierten Datensätze in chronologischer Reihenfolge bereitgestellt wird.

18. Verfahren nach einem der Ansprüche 13 bis 17, bei dem ausgewählt werden kann, ob die akustische Ausgabe chronologisch aufsteigend oder absteigend erfolgt.

19. Verfahren nach einem der Ansprüche 13 bis 18, bei dem eine Sprungfunktion aktiviert werden kann, mit der Datensätze mit zeitlichen Abständen selektierbar sind.

20. Verfahren nach Anspruch 19, bei dem den Datensätzen bei aktivierter Sprungfunktion ein akustisches Signal vorangestellt wird, das sich von dem ersten akustischen Signal unterscheidet.

21. Verfahren nach einem der Ansprüche 13 bis 20, wobei die Datensätze jeweils mehrere Datenfelder aufweisen und ein Datenfeld ein Datum und ein anderes Datenfeld einen Glucosewert beinhaltet, und anschließend Auswahlmöglichkeiten für eine zweite, medizinisch sinnvolle Selektion in den durch die erste Selektion selektierten Datensätzen bereitgestellt werden und eine zweite Selektion entsprechend einer vorgenommen Auswahl aus den Auswahlmöglichkeiten durchgeführt wird.

22. Computerprogramm mit Programmcode, der dazu geeignet ist, ein Verfahren nach einem der Ansprüche 13 bis 21 auszuführen, wenn das Computerprogramm auf einer Recheneinrichtung abläuft.

23. Computerprogramm nach Anspruch 22, das auf einem computerlesbaren Medium gespeichert ist.

24. Sprachausgabegerät mit einer Datenverarbeitungsvorrichtung nach einem der Ansprüche 1 bis 12.

## Claims

1. Data processing apparatus for processing measured values from a medical measuring device, having an input unit (18), a data input interface for communicating with an associated measuring device, a storage unit (14, 16) for storing data records which are input via the input unit (18) and/or are received via the data input interface, and a computing unit (12), wherein the data processing apparatus (10) is configured and set up in such a manner that a user can use the input unit (18) to trigger a first selection of data records from the data records stored in the storage unit (14, 16), **characterized in that** the data processing apparatus (10) also has an acoustic data output interface (22) and is configured and set up in such a manner that the selected data records are automatically output acoustically via the acoustic data output interface (22) after a selection has been made or after a corresponding button has been actuated by the user, and **in that** the acoustic output is effected in such a manner that, in order to differentiate from accompanying data records before or after the hit, each selected data record is preceded by a first acoustic signal during the acoustic output, and wherein an acoustic signal is not prefixed to the accompanying data records during the output or a second acoustic signal which differs from the first acoustic signal is prefixed.

2. Data processing apparatus according to Claim 1, in which the acoustic data output interface (22) is a loudspeaker and/or a connection for headphones or earphones.

3. Data processing apparatus according to Claim 1 or 2, in which the input unit (18) comprises a forward button and a back button (32) for controlling a virtual cursor, a selection button (46) or a numerical keypad (24).

4. Data processing apparatus according to one of Claims 1 to 3, in which the data input interface is a wireless interface for communicating with a measuring device.

5. Data processing apparatus according to one of Claims 1 to 3, which is formed in one piece with a measuring device via the data input interface.

6. Data processing apparatus according to one of Claims 1 to 5, in which at least the first selection of data records is a selection according to contents of a data record field, and a chronological selection which is effected by actuating an associated button of the input unit (18), or/and a keyword selection which is effected by inputting a code number which is representative of a keyword forming a selection criterion, or/and a range selection which is effected by inputting range limits for a data record field in which the selection is intended to be effected.

7. Data processing apparatus according to one of Claims 1 to 6, in which the selection possibilities for a second selection comprise a calculation of an average value from the data records determined by the first selection, or/and an amount of insulin administered within a particular period, or/and extreme values of the glucose value within the data records determined by the first selection, or/and a number of the hypoglycaemia or hyperglycaemia which have occurred within a particular period after or before the data records determined by the first selection, or/and particular events linked to the data records determined by the first selection, or/and the output of accompanying data records.

8. Data processing apparatus according to one of Claims 1 to 7, in which the selected data records are acoustically output both after the first selection and after the second selection, and an acoustic signal precedes each selected data record during the acoustic output.

9. Data processing apparatus according to one of Claims 1 to 8, wherein the user can use the input unit (18) to scroll through the selected data records.

10. Data processing apparatus according to one of Claims 1 to 9, wherein a skip function is provided and is used to select data records with intervals of time.

11. Data processing apparatus according to Claim 10, wherein an acoustic signal which differs from the first acoustic signal is prefixed to the data records when the skip function is activated.

12. Data processing apparatus according to one of Claims 1 to 11, wherein the data records each have a plurality of data fields, at least one data field of which comprises a glucose measured value, and wherein the data processing apparatus (10) is configured and set up in such a manner that, in response to the first selection, the data processing apparatus (10) provides the user with selection possibilities for a medically useful second selection of data records from the data records determined by the first selection.

13. Method for operating a data processing apparatus for processing measured values from a measuring device, according to which a first selection from data records stored in the data processing apparatus (10) is effected and the selected data records are output acoustically via an acoustic data output interface (22) in such a manner that the selected data records are automatically output acoustically via the acoustic data output interface (22) after a selection has been made or after a corresponding button has been actuated by the user, and the acoustic output is effected in such a manner that, in order to differentiate from accompanying data records before or after the hit, a first acoustic signal is prefixed to each selected data record during the acoustic output and an acoustic signal is not prefixed to the accompanying data records during the output or a second acoustic signal which differs from the first acoustic signal is prefixed.

14. Method according to Claim 13, in which the acoustic data output interface (22) is a loudspeaker and/or a connection for headphones or earphones.

15. Method according to one of Claims 13 to 14, in which at least the first selection of data records is effected according to contents of a data record field, and in which at least the first selection is a chronological selection which is effected by actuating an associated button of the input interface, or/and a keyword selection which is effected by inputting a code number which is representative of a keyword forming a selection criterion, or/and a range selection which is effected by inputting range limits for a data record field in which the selection is intended to be effected.

16. Method according to one of Claims 13 to 15, in which the selection possibilities for a second selection comprise a calculation of an average value from the data records determined by the first selection, or/and an amount of insulin administered within a particular period, or/and extreme values of the glucose value within the data records determined by the first selection, or/and a number of the hypoglycaemia or hyperglycaemia which have occurred within a particular period after or before the data records determined by the first selection, or/and particular events linked to the data records determined by the first selection, or/and the output of accompanying data records.

17. Method according to one of Claims 13 to 16, by means of which scrolling through the selected data records in chronological order is provided.

18. Method according to one of Claims 13 to 17, in which it is possible to select whether the acoustic output is effected in chronologically ascending or descending order.

19. Method according to one of Claims 13 to 18, in which it is possible to activate a skip function which can be used to select data records with intervals of time.

20. Method according to Claim 19, in which an acoustic signal which differs from the first acoustic signal is prefixed to the data records when the skip function is activated.

21. Method according to one of Claims 13 to 20, wherein the data records each have a plurality of data fields and one data field comprises a date and another data field comprises a glucose value, and selection possibilities for a second, medically useful selection in the data records selected by the first selection are then provided, and a second selection is carried out according to a selection made from the selection possibilities.

22. Computer program with program code which is suitable for carrying out a method according to one of Claims 13 to 21 when the computer program runs on a computing device.

23. Computer program according to Claim 22, which is stored on a computer-readable medium.

24. Voice output device having a data processing apparatus according to one of Claims 1 to 12.

## Revendications

1. Dispositif de traitement de données pour le traitement de valeurs de mesure d'un appareil de mesure médical, comportant une unité d'entrée (18), une interface d'entrée de données pour la communication avec un appareil de mesure associé, une unité de mémoire (14, 16) pour mémoriser des jeux de données entrés par l'intermédiaire de l'unité d'entrée (18) et/ou reçus par l'intermédiaire de l'interface d'entrée de données, et une unité de calcul (12), le dispositif de traitement de données (10) étant conçu et configuré de telle sorte qu'un utilisateur peut déclencher, au moyen de l'unité d'entrée (18), une première sélection de jeux de données parmi les jeux de données mémorisés dans l'unité de mémoire (14, 16),
**caractérisé en ce que**
le dispositif de traitement de données (10) comprend en outre une interface acoustique de sortie de données (22) et est conçu et configuré de telle sorte qu'une sortie des jeux de données sélectionnés s'effectue automatiquement après que la sélection a été effectuée ou après que l'utilisateur a actionné une touche correspondante, de manière acoustique via l'interface acoustique de sortie de données (22), et que
la sortie acoustique s'effectue de telle sorte que chaque jeu de données sélectionné est précédé d'un premier signal acoustique lors de la sortie acoustique afin de le délimiter des jeux de données d'accompagnement qui se trouvent avant ou après la concordance, et
les jeux de données d'accompagnement ne sont pas précédés d'un signal acoustique lors de la sortie ou sont précédés d'un deuxième signal acoustique qui diffère du premier signal acoustique.

2. Dispositif de traitement de données selon la revendication 1,
dans lequel
l'interface acoustique de sortie de données (22) est un haut-parleur et/ou un connecteur pour un casque ou un écouteur.

3. Dispositif de traitement de données selon la revendication 1 ou 2,
dans lequel
l'unité d'entrée (18) comprend une touche vers l'avant et une touche vers l'arrière (32) pour commander un curseur virtuel, une touche de sélection (46) ou un clavier numérique (24).

4. Dispositif de traitement de données selon l'une des revendications 1 à 3,
dans lequel
l'interface d'entrée de données est une interface sans fil pour la communication avec un appareil de mesure.

5. Dispositif de traitement de données selon l'une des revendications 1 à 3,
dans lequel
l'interface d'entrée de données est réalisée d'un seul tenant avec un appareil de mesure.

6. Dispositif de traitement de données selon l'une des revendications 1 à 5,
dans lequel
au moins la première sélection de jeux de données est une sélection en fonction du contenu d'un champ de jeu de données et une sélection chronologique qui est effectuée en actionnant une touche associée de l'unité d'entrée (18), et/ou une sélection par mots-clés qui est effectuée en entrant un numéro de code représentant un mot-clé formant un critère de sélection, et/ou une sélection d'intervalle qui est effectuée en entrant des limites d'intervalle pour un champ de jeu de données dans lequel doit se faire la sélection.

7. Dispositif de traitement de données selon l'une des revendications 1 à 6,
dans lequel
les possibilités de choix pour une deuxième sélection comprennent un calcul d'une valeur moyenne à partir des jeux de données déterminés par la première sélection, et/ou une quantité d'insuline administrée dans une période de temps définie, et/ou des valeurs extrêmes de glucose à l'intérieur des jeux de données déterminés par la première sélection, et/ou un certain nombre d'hypo- ou d'hyperglycémies survenant après ou avant les jeux de données déterminés par la première sélection dans une période de temps définie, et/ou des événements associés aux jeux de données déterminés par la première sélection, et/ou la sortie de jeux de données d'accompagnement.

8. Dispositif de traitement de données selon l'une des revendications 1 à 7, dans lequel
une sortie acoustique des jeux de données sélectionnés s'effectue aussi bien après la première sélection qu'après la deuxième sélection, et chaque jeu de données sélectionné est précédé d'un signal acoustique lors de la sortie acoustique.

9. Dispositif de traitement de données selon l'une des revendications 1 à 8,
dans lequel
l'utilisateur peut faire défiler les jeux de données sélectionnés à l'aide de l'unité d'entrée (18).

10. Dispositif de traitement de données selon l'une des revendications 1 à 9,
dans lequel
il est prévu une fonction de saut permettant de sélectionner des jeux de données avec des intervalles de temps.

11. Dispositif de traitement de données selon la revendication 10,
dans lequel
lorsque la fonction de saut est activée, les jeux de données sont précédés d'un signal acoustique qui diffère du premier signal acoustique.

12. Dispositif de traitement de données selon l'une des revendications 1 à 11,
dans lequel
les jeux de données comportent chacun plusieurs champs de données, dont au moins un champ de données contient une valeur de mesure du glucose, et
le dispositif de traitement de données (10) est conçu et configuré de telle sorte que le dispositif de traitement de données (10) offre à l'utilisateur, en réponse à la première sélection, des possibilités de choix pour une deuxième sélection médicalement raisonnable de jeux de données parmi les jeux de données déterminés par la première sélection.

13. Procédé pour faire fonctionner un dispositif de traitement de données pour le traitement de valeurs de mesure d'un appareil de mesure médical, dans lequel
une première sélection s'effectue parmi des jeux de données mémorisés dans le dispositif de traitement de données (10), et une sortie des jeux de données sélectionnés s'effectue par voie acoustique via une interface acoustique de sortie de données (22), de telle sorte qu'une sortie des jeux de données sélectionnés s'effectue automatiquement après que la sélection a été effectuée ou après que l'utilisateur a actionné une touche correspondante, de manière acoustique via l'interface acoustique de sortie de données (22), et que
la sortie acoustique s'effectue de telle sorte que chaque jeu de données sélectionné est précédé d'un premier signal acoustique lors de la sortie acoustique afin de le délimiter des jeux de données d'accompagnement qui se trouvent avant ou après la concordance, et
les jeux de données d'accompagnement ne sont pas précédés d'un signal acoustique lors de la sortie ou sont précédés d'un deuxième signal acoustique qui diffère du premier signal acoustique.

14. Procédé selon la revendication 13,
dans lequel
l'interface acoustique de sortie de données (22) est un haut-parleur et/ou un connecteur pour un casque ou un écouteur.

15. Procédé selon l'une des revendications 13 à 14,
dans lequel
au moins la première sélection de jeux de données est une sélection en fonction du contenu d'un champ de jeu de données, et ladite première sélection est une sélection chronologique qui est effectuée en actionnant une touche associée de l'interface d'entrée, et/ou une sélection par mots-clés qui est effectuée en entrant un numéro de code représentant un mot-clé formant un critère de sélection, et/ou une sélection d'intervalle qui est effectuée en entrant des limites d'intervalle pour un champ de jeu de données dans lequel doit se faire la sélection.

16. Procédé selon l'une des revendications 13 à 15,
dans lequel
les possibilités de choix pour une deuxième sélection comprennent un calcul d'une valeur moyenne à partir des jeux de données déterminés par la première sélection, et/ou une quantité d'insuline administrée dans une période de temps définie, ou/ou des valeurs extrêmes de glucose à l'intérieur des jeux de données déterminés par la première sélection, et/ou un certain nombre d'hypo- ou d'hyperglycémies survenant après ou avant les jeux de données déterminés par la première sélection dans une période de temps définie, et/ou des événements associés aux jeux de données déterminés par la première sélection, et/ou la sortie de jeux de données d'accompagnement.

17. Procédé selon les revendications 13 à 16, permettant de faire défiler les jeux de données sélectionnés dans l'ordre chronologique.

18. Procédé selon l'une des revendications 13 à 17, permettant de choisir si la sortie acoustique est chronologiquement ascendante ou descendante.

19. Procédé selon l'une des revendications 13 à 18, permettant d'activer une fonction de saut permettant de sélectionner des jeux de données avec des intervalles de temps.

20. Procédé selon la revendication 19,
dans lequel
lorsque la fonction de saut est activée, les jeux de données sont précédés d'un signal acoustique qui diffère du premier signal acoustique.

21. Procédé selon l'une des revendications 13 à 20,
dans lequel
les jeux de données présentent chacun plusieurs champs de données, et un champ de données contient une date et un autre champ de données contient une valeur de glucose, et ensuite des possibilités de choix sont fournies pour une deuxième sélection médicalement raisonnable dans les jeux de données sélectionnés par la première sélection, et une deuxième sélection est effectuée en correspondance d'un choix effectué parmi les possibilités de choix.

22. Programme d'ordinateur ayant un code de programme qui est apte à mettre en œuvre un procédé selon l'une des revendications 13 à 21 lorsque le programme d'ordinateur est exécuté sur un dispositif de calcul.

23. Programme d'ordinateur selon la revendication 22 qui est stocké sur un support lisible à l'ordinateur.

24. Dispositif de sortie vocale comportant un dispositif de traitement de données selon l'une des revendications 1 à 12.
